# EUROPÄISCHE PATENTANMELDUNG

(11) **EP 3 739 093 A1**
(43) Veröffentlichungstag der Anmeldung: **18.11.2020**
(21) Anmeldenummer: 20181840.8
(22) Anmeldetag: 23.07.2019
(51) Int. Cl.: D03D 1/00, A41D 31/26, A41D 1/00, A61B 5/00

(54) **VERFAHREN ZUM HERSTELLEN EINES TEXTILSTÜCKES**

(30) Priorität: 23.08.2018 DE 102018120619
(62) Teilanmeldung aus: 19744672.7
(71) Anmelder: B-Horizon GmbH, 93161 Sinzing (DE)
(72) Erfinder: Kabany, Mohammad, 93053 Regensburg (DE)
(74) Vertreter: Peters, Andreas

(57) **Zusammenfassung**

Die Erfindung betrifft ein Verfahren zum Herstellen eines Textilstückes, insbesondere eines Kleidungsstückes, umfassend die folgenden Schritte: Bereitstellen einer Vorgabeeinheit, welche eine Form und/oder Größe und/oder einen Textilstoff vorgibt, Bereitstellen des Textilstoffes, welcher mit einem Textilbasisstoff gebildet ist, wobei in einem nächsten Schritt mittels einer Produktionseinheit der Textilbasisstoff in zumindest zwei räumlich voneinander unabhängige und durch zumindest eine Schneidmarkierung getrennte Textilsegmente unterteilt wird, wobei die Textilsegmente nach deren Zusammenfügung das durch die Vorgabeeinheit vorgegebene Textilstück, insbesondere ein Kleidungsstück, ergeben, wobei in einem nächsten Schritt elektrisch leitfähige Fäden in die einzelnen Textilsegmente durch entsprechende Vorgabeparameter der Vorgabeeinheit eingearbeitet werden, wobei die elektrisch leitfähigen Fäden die Schneidmarkierung nicht kreuzen und auch nicht zumindest stellenweise auf ihr verlaufen.

## Beschreibung

Die vorliegende Erfindung betrifft ein Verfahren zum Herstellen eines Textilstückes, insbesondere eines Kleidungsstückes, sowie eine Vorrichtung zur Herstellung eines Textilstückes, insbesondere eines Kleidungsstückes gemäß den jeweiligen Oberbegriffen der Patentansprüche 1 und 10.

Ein Kernelement des vorliegenden Verfahrens ist in einem ersten Schritt zunächst das Bereitstellen einer Vorgabeeinheit, welche eine Form und/oder eine Größe und/oder einen Textilstoff bereitstellt. Dabei kann es sich bei einem "Bereitstellen" im Sinne der vorliegenden Erfindung, um eine tatsächlich haptisch strukturelle Bereitstellung eines haptisch strukturell wahrnehmbaren Textilstoffes handeln, oder aber der Textilstoff liegt lediglich virtuell, das heißt in datentechnischer Form, vor. Zum Beispiel kann ein derartiger Textilstoff datentechnisch durch verschiedene Datenpunkte erfasst sein, welche in der Vorgabeeinheit hinterlegt sind. Beispielsweise besteht eine derartige Hinterlegung im Rahmen eines Datenbankspeicherungssystems.

In einem nächsten Schritt wird ein Textilstoff bereitgestellt, welcher mit einem Textilbasisstoff gebildet ist.

Dieser Schritt kann heißen, dass zunächst entweder strukturell oder virtuell der Textilstoff, zum Beispiel auch zusammen mit den elektrisch leitfähigen Fäden, gewebt wird. Dies würde dann heißen, dass die elektrisch leitfähigen Fäden nicht erst nachträglich in den Textilbasisstoff eingebracht werden, sondern bereits während des Webprozesses in den Textilbasisstoff eingewoben werden. In diesem Fall können die elektrisch leitfähigen Fäden einzelne Fäden oder Fädenabschnitte des eigentlichen Textilstoffes ersetzen.

Auch hierbei kann der Textilstoff entweder haptisch strukturell bereitgestellt werden oder aber lediglich virtuell in Form von Daten in der Vorgabeeinheit hinterlegt sein und/oder in die Vorgabeeinrichtung hineingeladen werden.

Ein Kernelement der vorliegenden Erfindung ist, dass in einem nächsten Schritt mittels einer Produktionseinheit der Textilbasisstoff in zumindest zwei räumlich voneinander unabhängige und durch zumindest eine Schneidmarkierung getrennte Textilsegmente unterteilt wird, wobei die Textilsegmente nach deren Zusammenfügung das durch die Vorgabeeinheit vorgegebene Textilstück, insbesondere ein Kleidungsstück, ergeben, wobei in einem nächsten Schritt elektrische Leitfäden in die einzelnen Textilsegmente durch entsprechende Vorgabeparameter der Vorgabeeinheit eingearbeitet werden, wobei die elektrisch leitfähigen Fäden die Schneidmarkierung nicht kreuzen und auch nicht zumindest stellenweise auf ihr verlaufen.

Mit anderen Worten wird eine derartige Schneidmarkierung an keiner Stelle geschnitten, sodass das gesamte Verfahren auf ungeschnittenen elektrischen Leitfäden beruht. Eine Durchschneidung der elektrischen Leitfäden hat nämlich ergeben, dass an dessen durchschnittenen Ende sich Materialfehler und besondere Hitzepunkte bei der Benutzung des elektrischen Leitfadens ergeben.

Vorzugsweise verläuft zumindest eine Schneidmarkierung zumindest teilweise durch den, vorzugweise flächigen, Textilstoff hindurch. Beispielsweise weist jedes Textilsegment als Teil eines Begrenzungsrands zumindest eine Schneidmarkierung auf.

Zum Beispiel weist jeder elektrisch leitfähige Faden einen Abstand von mindestens zwei Millimetern von jeder Schneidmarkierung auf. Dies stellt sicher, dass während des Schneideprozesses der elektrisch leitfähige Faden nicht in Mitleidenschaft gezogen wird.

Weiter zum Beispiel weist ein Textilsegment mindestens zwei, vorzugsweise parallel zueinander, verlaufende elektrisch leitfähige Fäden auf. Ein Abstand dieser Fäden kann mindestens einen Millimeter betragen. Die Bahnen der elektrischen Fäden können jedoch auch gekrümmt, kreisförmig oder ellipsoid verlaufen.

Zum Beispiel weisen zumindest zwei Teilsegmente elektrisch leitfähige Fäden unterschiedlicher Länge auf. Dabei können diese Fäden innerhalb eines Teilsegments jeweils unterschiedliche Längen aufweisen und/oder Fäden unterschiedlicher Teilsegmente unterschiedliche Längen aufweisen.

Mit anderen Worten wird in dem oben genannten Verfahren zunächst mittels der Produktionseinheit ein Schnittmuster auf einen vorzugsweise flächigen Textilstoff aufgebracht und/ oder mittels eines virtuellen Musters wird ein derartiger Textilstoff virtuell überlagert (zum Beispiel ist so das Muster über den Textilstoff, beispielsweise nur, virtuell darüber gelegt), wobei dieses Schnittmuster im Anschluss, entsprechend der in dem Schnittmuster hinterlegten Schnittlinien, zerschnitten wird, sodass die dann zerschnittenen Textilsegmente im Nachgang zusammengefügt werden können, um dann ein hier beanspruchtes Textilstück zu ergeben.

Die elektrisch leitfähigen Fäden dienen zur Kontaktierung von elektrisch betriebenen Sensoren und/oder anderen elektrisch zu betriebenen Elementen. Allgemein kann es sich bei diesen Elementen auch von einer oder mehreren Vorrichtungen zur Messung von Druck und/oder Feuchtigkeit handeln.

Es ist möglich, dass diese Sensoren noch vor dem Zerschneiden entlang der Schneidlinien auf die Textilsegmente, zumindest teilweise aufgebracht, beispielsweise aufgedruckt, und mit den elektrisch leitfähigen Fäden in elektrisch leitendem Kontakt gebracht werden. Nach dem Zusammenfügen der einzelnen Teilsegmente zu dem Textilstoff, also zum Beispiel dem Kleidungsstück, können freie Enden der elektrisch leitfähigen Fäden mit einer Energieversorgungseinheit elektrisch leitend verbunden werden.

Alternativ hierzu können jedoch auch die elektrisch betriebenen Sensoren und/oder anderen elektrisch zu betriebenen Elemente erst nach der Entstehung des Textilstückes, also zum Beispiel des Kleidungsstückes, auf dieses aufgebracht werden.

Die unter anderem auch hier beschriebene Vorrichtung zur Messung von Druck und/oder Feuchtigkeit umfasst zumindest einen Sensor zur Messung von Druck und/oder Feuchtigkeit, wobei der Sensor zumindest einen Kondensator mit zumindest zwei Elektroden umfasst, welche, insbesondere in einer horizontalen Richtung, entlang eines und auf einem, insbesondere flexiblen, Trägermaterial zueinander angeordnet sind, wobei zwischen den Elektroden zumindest eine dielektrische Schicht angeordnet ist.

Die horizontale Richtung ist vorzugsweise eine Haupterstreckungsrichtung des flexiblen Trägermaterials. Bei dem Trägermaterial kann es sich um den Textilbasisstoff handeln.

"Flexibel" heißt in diesem Zusammenhang, dass das Trägermaterial zumindest stellenweise biegsam und damit elastisch ist.

Insbesondere kann es sich bei dem Trägermaterial um einen Webstoff oder um einen sonstigen Bekleidungsstoff, wie zum Beispiel ein Polyester, handeln.

Die dielektrische Schicht beabstandet damit die beiden Elektroden in einer horizontalen und/oder in einer dazu senkrechten Querrichtung.

Zum Beispiel ist auf einer dem Trägermaterial abgewandten Seite zumindest eine Elektrode und/oder dielektrische Schicht zumindest stellenweise, zumindest eine, zumindest teilweise flüssigkeitsdurchlässige und/oder flüssigkeitsabsorbierende Feuchteschicht angeordnet, wobei somit die zumindest eine Elektrode und/oder dielektrische Schicht in einer Querrichtung zwischen dem Trägermaterial und der Feuchteschicht angeordnet sind, sodass eine Kapazität durch die auf die dielektrische Schicht zumindest teilweise treffende Flüssigkeit, zumindest teilweise verändert, wobei eine Verarbeitungseinheit dazu eingerichtet und dafür vorgesehen ist diese Änderung zu messen und/oder zu speichern, sodass ein kapazitiver Feuchtesensor entsteht.

Ein kapazitiver Feuchtesensor ist im Prinzip ein Kondensator, dessen Dielektrikum vorzugsweise aus einem hygroskopischen Polymerschicht besteht, die entsprechend der Feuchtigkeit der Umgebungsluft Feuchtigkeit aufnimmt (absorbiert) oder abgibt (desorbiert) bis ein Gleichgewichtszustand (Diffusionsgefälle ist = 0) erreicht ist. Dabei verändert sich die Dielektrizitätskonstante des Polymermaterials als Funktion eines Feuchtegehalts.

Die Aufgabe der Verarbeitungseinheit besteht unter anderem darin, vorzugsweise auch aus einer gemessenen Umgebungstemperatur und dem feuchtigkeitsabhängigen Kapazitätswert des Sensors die relative Feuchte möglichst genau zu ermitteln.

Gemäß zumindest einer Ausführungsform umfasst die Vorrichtung zur Messung von Druck und/oder Feuchtigkeit zumindest einen Sensor zur Messung von Druck und/oder Feuchtigkeit, wobei der Sensor zumindest einen Kondensator mit zumindest zwei Elektroden umfasst, welche insbesondere in einer horizontalen Richtung entlang eines und auf einem, insbesondere flexiblen, Trägermaterial zueinander angeordnet sind, wobei zwischen den Elektroden zumindest eine dielektrische Schicht angeordnet ist.

Zum Beispiel ist auf einer dem Trägermaterial abgewandten Seite zumindest eine Elektrode und/oder dielektrische Schicht, zumindest stellenweise, zumindest eine, zumindest teilweise flüssigkeitsdurchlässige und/oder flüssigkeitsabsorbierende Schicht (= Feuchteschicht) angeordnet, wobei somit die zumindest eine Elektrode und/oder dielektrische Schicht in Querrichtung zwischen dem Trägermaterial und der Feuchteschicht angeordnet sind, sodass eine Kapazität durch die auf die dielektrische Schicht zumindest teilweise treffende Flüssigkeit, zumindest teilweise verändert wird, wobei eine Verarbeitungseinheit dazu eingerichtet und dafür vorgesehen ist diese Änderung zu messen und/oder zu speichern, sodass ein kapazitiver Feuchtesensor entsteht.

Die Feuchteschicht kann mit einem dielektrischen Material gebildet sein. Das Material der Feuchteschicht kann verschieden von dem Material der wasserundurchlässigen Schicht sein.

Der Sensor und/oder die Verarbeitungseinheit können mittels einer Batterie oder einer Festnetzstromversorgung mit elektrischer Energie versorgt werden.

Alternativ oder zusätzlich ist die Erzeugung von elektrischer Energie zur Versorgung des Sensors und/oder Verarbeitungseinheit mittels sogenannten "Energy Harvesting" möglich.

Als Energy Harvesting (wörtlich übersetzt *Energie-Ernten*) bezeichnet man die Gewinnung kleiner Mengen von elektrischer Energie aus Quellen wie Umgebungstemperatur, Vibrationen oder Luftströmungen für mobile Geräte mit geringer Leistung. Die dafür eingesetzten Strukturen werden auch als Nanogenerator bezeichnet. Energy Harvesting vermeidet bei Drahtlostechnologien Einschränkungen durch kabelgebundene Stromversorgung oder Batterien.

Möglichkeiten des Energy Harvesting:
- Piezoelektrische Kristalle erzeugen bei Krafteinwirkung, beispielsweise durch Druck oder Vibration, elektrische Spannungen. Diese Kristalle können an oder auf dem Trägermaterial angeordnet sein.
- Thermoelektrische Generatoren und pyroelektrische Kristalle gewinnen aus Temperaturunterschieden elektrische Energie. Diese Generatoren können an oder auf dem Trägermaterial angeordnet sein.
- Über Antennen kann die Energie von Radiowellen, eine Form von elektromagnetischer Strahlung, aufgefangen und energetisch verwendet werden. Ein Beispiel dafür sind die passiven RFIDs. Diese Antennen können an oder auf dem Trägermaterial angeordnet sein.
- Photovoltaik, elektrische Energie aus der Umgebungsbeleuchtung.
- Osmose.

Gemäß zumindest einer Ausführungsform ist der Sensor zusätzlich ein kapazitiver Drucksensor, wobei die Verarbeitungseinheit zusätzlich dazu eingerichtet und dafür vorgesehen ist, eine durch äußeren Druck verursachte Kapazitätsänderung des Kondensators zu messen und/oder zu speichern.

Grundsätzlich handelt es sich bei einem kapazitiven Sensor also um einen Sensor, welcher auf Basis der Veränderung der elektrischen Kapazität eines einzelnen Kondensators oder eines Kondensatorsystems arbeitet. Die Beeinflussung der Kapazität durch die zu erfassende Größe kann dabei auf verschiedene Arten erfolgen, die primär durch den Verwendungszweck bestimmt ist.

Ein kapazitiver Sensor basiert unter anderem darauf, dass zwei Elektroden, einer davon kann die zumessende Oberfläche sein, die "Platten" eines elektrischen Kondensators bilden, dessen Kapazität oder Kapazitätsänderung gemessen wird, die folgendermaßen beeinflusst werden kann:
- Eine Platte wird durch den zumessenden Effekt verschoben und/oder verformt, wodurch sich der Plattenabstand und damit die elektrische messbare Kapazität ändern.
- Die Platten sind starr und die Kapazität an Sich ändert sich dadurch, dass ein elektrisch leitendes Material oder ein Dielektrikum in unmittelbare Nähe gebracht wird.
- Die wirksame Plattenfläche ändert sich, indem die Platten wie bei einem Drehkondensator gegeneinander verschoben werden.

Um auch kleine Veränderungen besser detektieren zu können kann die eigentliche Messelektrode häufig mit einer Schirmelektrode umgeben sein, die den inhomogenen Randbereich des elektrischen Feldes von der Messelektrode abschirmt, dadurch ergibt sich zwischen Messelektroden üblicherweise geerdeter Gegenelektrode eine annähernd paralleles elektrisches Feld mit der bekannten Charakteristik eines idealen Plattenkondensators.

Ein kapazitiver Drucksensor ist insbesondere ein solcher bei dem die Kapazitätsänderung infolge des Durchbiegens einer Membran und der resultierenden Änderung des Plattenabstands als Sensoreffekt ausgewertet wird. Zum Beispiel handelt es sich bei der Membran um das oben genannte Dielektrikum oder aber um die einzelnen Kondensatorelektroden, welche insbesondere in Form einer Platte ausgeführt sein können. Mit anderen Worten ist in einer derartigen Ausführungsform in neuartiger Art und Weise ein kapazitiver Feuchtesensor mit einem kapazitiven Drucksensor kombiniert, jedoch ohne dass diese Bauteile voneinander getrennte Elemente oder zwei separate Sensoren bildeten, sondern es handelt sich bei vorliegender Ausführungsform um ein "Two in One"-Konzept, in welchem der gleiche Sensor sowohl als Feuchtesensor, als auch als Drucksensor fungiert.

Gemäß zumindest einer Ausführungsform handelt es sich bei dem Trägermaterial um einen Webstoff, insbesondere in welchem elektrische Leiterbahnen zur elektrischen Kontaktierung des Sensors und der Verarbeitungseinheit eingewoben sind.

Bei einem Webstoff handelt es sich im Sinne der Erfindung daher um ein Gewebe, welches manuell oder maschinell auf Basis von einzelnen Fäden gewebt wurde.

Die elektrischen Leiterbahnen können in einem Gewebe daher zusätzlich neben den üblichen Fasern und Gewebesträngen integriert sein oder aber einzelne Gewebestränge, welche das Gewebenetz ausbilden ersetzen.

Je nach Abstand und Eigenschaften der einzelnen Fäden (hochgedreht, bauschig, usw.) können ganz lockere Gewebe, wie Verbandgewebe oder Dichtegewebe wie Brokatstoff, entstehen. Längselastisch werden Gewebe durch, als Kettenfäden eingesetzte Gummifäden (mehr Bändern verwendet) oder Kräusel- und Bauschgarne, verwendet. Sie werden gespannt, verarbeitet und ziehen sich im Ruhezustand zusammen. Bauschgarne bestehen aus texturierten, also gekräuselten synthetischen Fasern. Die Kräuselung verändert die Eigenschaften der synthetischen Fasern. Die darauf gesponnenen Garne sind sehr elastisch und voluminös und haben eine gute Wärmedämmung.

Zum Beispiel kann das Trägermaterial Teil eines Bezugstoffes eines Sitzes, insbesondere eines Fahrzeugsitzes oder eines Bürostuhls, sein. Insofern kann der Sensor, vorzugsweise jedoch die gesamte Vorrichtung, auf dem Bezugsstoff eines solchen Sitzes aufgebracht oder in einen solchen integriert sein.

Zum Beispiel ist die Verarbeitungseinheit dazu eingerichtet und dafür vorgesehen, die einzelnen Feuchte- sowie Druckwerte zu erfassen und aus einer Kombination der einzelnen Feuchte- und Druckwerte zumindest einen jeweiligen Kennwert zu ermitteln, aus welchem ableitbar ist, welches Individuum (mit Gewicht und/oder Größe) gerade den Fahrzeugsitz besetzt.

Zum Beispiel kann aus der Druckmessung durch die Verarbeitungseinheit ein Gewicht der jeweiligen Person abgeleitet und festgestellt werden. Auch kann die jeweilige Feuchtigkeit, welche die jeweilige Person an den Sensor abgibt, gemessen werden, wobei der jeweilige Kennwert zum Beispiel ein Produkt aus dem relativen Feuchtigkeitswert mal der von der Verarbeitungseinheit ermittelten Belastungsgewicht ist.

Überschreitet ein derartiger Kennwert einen entsprechenden Grenzwert kann die Verarbeitungseinheit insbesondere mittels einer Anbindung an die Elektronik des Fahrzeugs, eine Warnung aussprechen. Diese Warnung kann dahingehend lauten, dass der Sitz überbelegt ist oder der Fahrer zu stark schwitzt. Diese Warnung kann jedoch auch ersetzt werden durch eine entsprechende Anzeige dahingehend welcher Belegungstyp den Sitz nutzt. Bei einem Belegungstyp kann es sich um eine Gewichtsklassifikation eines jeweiligen Benutzers handeln, oder aber auch darum Handeln ob es sich bei dem Benutzer um ein Tier, einen Menschen oder auch um eine Sache handelt. Vorzugsweise ist daher die Verarbeitungseinheit in eine Anzeigenelektronik des Fahrzeugs integrierbar, zumindest jedoch mit einer solchen verbindbar.

Hierzu ist denkbar, dass die Verarbeitungseinheit sich zum Beispiel mittels Bluetooth oder einer sonstigen Wireless Verbindung mit einer Empfangseinheit des Fahrzeugs verbindet und der jeweilige Kenn- oder Grenzwert und/oder die jeweilige Warnung und/oder die jeweilige Identifikation des Benutzers auf einem Display des Fahrzeugs wiedergegeben werden.

Alternativ oder zusätzlich ist vorstellbar, dass diese einzelnen Werte und/oder Identifikationen auch extern abrufbar und/oder extern darstellbar sind. Zum Beispiel kann das Auto auf eine Überbelegung hin von einem externen Controller überwacht werden.

Zum Beispiel kann mittels einer Datenverbindung die Verarbeitungseinheit mit einer Auslöseinheit eines Airbags in Verbindung stehen, sodass die Verarbeitungseinheit auch die Auslöseinheit steuern und/oder regeln kann, insbesondere in Bezug auf einen Auslösezeitpunkt des Airbags. Zusätzlich und/oder alternativ ist es möglich, dass die Verarbeitungseinheit eine Controllereinheit des Airbags mit Daten zum Beispiel im Hinblick auf einen Belegungstypen, eine Position und/oder ein Gewicht eines Benutzers des Fahrzeugsitzes versorgt.

Diese Daten können dazu führen, dass der Auslösezeitpunkt und die Auslösereihenfolge des Airbags auf den Benutzer angepasst sind, sodass ein Personenschaden an dem Benutzer vermieden wird.

Gemäß zumindest einer Ausführungsform ist zumindest eine Elektrode und/oder dielektrische Schicht auf dem Trägermaterial oder auf einer auf dem Trägermaterial angeordneten, insbesondere wasserundurchlässigen Schicht aufgedruckt oder mittels eines Dünnschichtverfahrens aufgebracht.

Dies heißt, dass zumindest ein Element, vorzugsweise sowohl die Elektrode als auch die dielektrische Schicht, auf dem Trägermaterial oder auf einer, zwischen dem Sensor und dem Trägermaterial aufgebrachten, vorzugsweise elektrisch nicht leitfähigen, weiter vorzugsweise wasserundurchlässigen, Schicht mittels eines Druckverfahrens aufgedruckt sind.

Bei dem Druckverfahren kann es sich zum Beispiel um ein Inkjetverfahren handeln.

Zum Beispiel ist die Verarbeitungseinheit in der gleichen Weise wie der Sensor auf das Trägermaterial aufgebracht. Hierzu ist vorstellbar, dass auch die Verarbeitungseinheit, zumindest jedoch eine, insbesondere leitende, Schicht der Verarbeitungseinheit auf das Trägermaterial zum Beispiel aufgedruckt ist. Die Datenkommunikation zwischen der Verarbeitungseinheit und dem Sensor kann dann über die oben genannten Leiterbahnen entstehen. Diese Leiterbahnen können zumindest teilweise, vorzugsweise jedoch vollständig, in den Webstoff eingewoben sein oder sogar einzelne Fasern des Webstoffs selbst ausbilden.

Zum Beispiel ist zumindest eine Elektrode flächig ausgeführt. Das heißt, dass eine Dicke der Elektrode im Vergleich zu deren Flächenausdehnung vernachlässigbar ist. Eine solche Elektrode kann daher insbesondere mittels eines Druckverfahrens hergestellt werden.

Alternativ hierzu kann eine Dicke, zumindest einer Elektrode, höchstens 5 mm betragen. Hierzu kann das Druckverfahren mehrmals angewandt werden, sodass zumindest zwei, vorzugsweise jedoch dann mehr, Einzeldruckschichten übereinandergestapelt werden.

Des Weiteren kann die Elektrode auch mittels eines 3D-Druckverfahrens auf dem Trägermaterial angeordnet sein.

### 1. Das FDM-Verfahren (Fused Deposition Modeling)

### Alternativbezeichnungen: Fused Filament Fabrication (FFF), Fused Layer Modeling (FLM)

Das Verfahren bezeichnet schichtweises Auftragen (Extrusion) eines Materials durch eine heiße Düse. Das Verbrauchsmaterial befindet sich in Form eines langen Drahts (sog. Filament) auf einer Rolle und wird durch die Fördereinheit in einen Druckkopf geschoben, dort eingeschmolzen und auf einem Druckbett ausgebracht. Druckkopf und/oder Druckbett sind dabei in drei Richtungen beweglich. So können Kunststoffschichten schrittweise aufeinander aufgebracht werden.

### 2. Das SLS Verfahren (Selektives Lasersintern)

Im Unterschied zum Sinterverfahren, bei dem Stoffe in Pulverform unter Hitzeeinwirkung miteinander verbunden werden, geschieht dies beim SLS-Verfahren selektiv durch einen Laser (alternativ auch Elektronenstrahl oder Infrarotstrahl). Es wird also nur ein bestimmter Teil des Pulvers miteinander verschmolzen.

Dazu wird stets eine dünne Pulverschicht von der Beschichtungseinheit auf dem Druckbett ausgebracht. Der Laser (oder andere Energiequelle) wird nun punktgenau auf einzelne Stellen der Pulverschicht ausgerichtet, um die erste Schicht der Druckdaten auszubilden. Hierbei wird das Pulver an- oder aufgeschmolzen und verfestigt sich anschließend wieder durch geringfügiges Abkühlen. Das nicht aufgeschmolzene Pulver bleibt um die gesinterten Bereiche herum liegen und dient als Stützmaterial. Nachdem eine Schicht verfestigt ist, senkt sich das Druckbett um den Bruchteil eines Millimeters ab. Die Beschichtungseinheit fährt nun über das Druckbett und bringt die nächste Pulverschicht aus. Anschließend wird die zweite Schicht der Druckdaten durch den Laser (oder eine andere Energiequelle) gesintert. So entsteht schichtweise ein dreidimensionales Objekt.

### 3. Three-Dimensional Printing (3DP)

Das 3DP-Verfahren funktioniert sehr ähnlich wie das selektive Lasersintern, doch anstelle einer gerichteten Energiequelle verfährt ein Druckkopf über das Pulver. Dieser gibt winzige Tröpfchen von Bindemittel auf die zugrunde liegenden Pulverschichten ab, die so miteinander verbunden werden. Ansonsten ist dieses Verfahren dem SLS-Verfahren gleich.

### 4. Stereolithographie (SLA)

Anstelle eines Kunststoffdrahts oder Druckmaterials in Pulverform kommen beim Stereolithographie-Verfahren flüssige Harze, sog. Photopolymere, zum Einsatz. Sie werden schichtweise durch UV-Strahlung verhärtet und erzeugen so dreidimensionale Objekte. Dafür wird die Bauplattform im Harzbecken schrittweise abgesenkt. Es gibt auch Varianten (sog. Polyjet-Verfahren) ohne ein ganzes Becken mit flüssigem Harz. Dafür wird ein Epoxidharz tröpfchenweise aus einer Düse aufgebracht und durch einen UV-Laser sofort ausgehärtet.

### 5. Laminated Object Manufacturing (LOM)

### Alternativbezeichnung: Layer Laminated Manufacturing (LLM)

Das Verfahren basiert weder auf chemischen Reaktionen, noch auf einem thermischen Prozess. Es wird dabei mit einem trennenden Werkzeug (z.B. einem Messer oder Kohlendioxidlaser), einer Folie oder einer Platte (z.B. Papier) an der Kontur geschnitten und schichtweise aufeinander geklebt. So entsteht durch Absenken der Bauplattform ein Schichtobjekt aus geklebten, übereinanderliegenden Folien.

Eine oder mehrere wasserundurchlässige Schichten und/oder auch die Feuchteschicht können in derselben Art und/oder Dicke wie die Elektrode aufgebracht werden.

Gemäß zumindest einer Ausführungsform bedeckt die Feuchteschicht den Kondensator vollständig.

Dies kann heißen, dass die Feuchteschicht, nach außen, das heißt in der Querrichtung den Sensor nach außen abgrenzt und abschließt, sodass der Sensor zwischen der Feuchteschicht und dem Trägermaterial angeordnet ist.

Gemäß zumindest einer Ausführungsform weist der Sensor zumindest einen weiteren Kondensator auf, welcher in der Querrichtung unter oder über dem Kondensator angeordnet und durch eine weitere wasserundurchlässige Schicht beabstandet von dem Kondensator auf oder unter dieser weiteren wasserundurchlässigen Schicht angeordnet ist, sodass ein Kondensatorenstack entsteht.

Der weitere Kondensator kann in der gleichen Weise wie der Kondensator aufgebaut sein und ebenso in einer gleichen Weise wie der Kondensator auf die weitere wasserundurchlässige Schicht angeordnet sein.

Mittels eines derartigen Kondensatorenstacks kann die Sensorik ganz besonders einfach verfeinert werden nämlich insofern, als dass denkbar ist das bei zwei den Kondensatorstack ausbildenden Sensoren beide Sensoren die gleichen Aufgaben verrichten, jedoch durch die einzelnen Sensoren jeweilige Messwerte ermittelt werden, die zusammen genommen auf einen Mittelwert schließen lassen. Zum Beispiel wird von jedem der beiden Sensoren jeweils die (relative) Feuchtigkeit der Umgebung gemessen wobei aus diesen beiden Messwerten dann der Feuchtigkeitsmittelwert ermittelt wird. Gleiches kann entsprechend mit der Druckmessung geschehen, sodass die Genauigkeit der gesamten Messung insbesondere einer Kombination der Messungen von (relativer) Feuchtigkeit und dem jeweiligen Druck besonders genau ausgestaltet werden kann.

Gemäß zumindest einer Ausführungsform bildet die wasserundurchlässige Schicht und/oder die weitere wasserundurchlässige Schicht die dielektrische Sicht zumindest teilweise selbst aus.

Dies kann heißen, dass anstatt der separaten Positionierung einer dielektrischen Schicht neben der wasserundurchlässigen Schicht und/oder neben der weiteren wasserundurchlässigen Schicht, diese dielektrische Schicht selbst durch die wasserundurchlässige Schicht und/oder die weitere wasserundurchlässige Schicht gebildet ist.

Eine derartige Erzeugung der dielektrischen Schicht durch die wasserundurchlässigen Schicht(en) bildet daher ein besonders einfaches und kostengünstiges Herstellungsverfahren zu einer kostengünstigen Vorrichtung.

Davon abgesehen kann grundsätzlich vorgesehen sein die Elektroden, die dielektrische Schicht und die wasserundurchlässige Schicht(en) derart zueinander anzuordnen, dass ein elektrischer Kurzschluss in jedem Fall verhindert ist.

Gemäß zumindest einer Ausführungsform beträgt eine maximale Dicke der Feuchteschicht wenigstens 30 % und höchstens 80 % der maximalen Dicke der wasserundurchlässigen Schicht und/oder der maximalen Dicke der weiteren wasserundurchlässigen Schicht.

Dies stellt nicht nur einen besonders flach gebauten Sensor sicher, sondern gewährleistet auch eine besonders schnelle Reaktionszeit auf Feuchtigkeitsveränderungen. Die von außen auf die Feuchteschicht einwirkende Feuchtigkeit muss daher keine großen Strecken zu dem Dielektrikum durchwandern.

Des Weiteren betrifft die vorliegende Erfindung ein Verfahren zur Messung von Druck und/oder Feuchtigkeit wobei insbesondere angemerkt sei, dass alle für die obig beschriebene Vorrichtung offenbarten Merkmale auch für das hier beschriebene Verfahren offenbart sind und umgekehrt.

Gemäß zumindest einer Ausführungsform umfasst das Verfahren zu Messung von Druck und/oder Feuchtigkeit zunächst einen ersten Schritt mittels welchem zumindest ein Sensor zu Messung von Druck und/oder Feuchtigkeit bereitgestellt wird, wobei der Sensor zumindest einen Kondensator mit zumindest zwei Elektroden, welche, insbesondere in einer horizontalen Richtung entlang eines und auf einem, insbesondere flexiblem, Trägermaterial zueinander angeordnet sind aufweist, wobei zwischen den Elektroden zumindest eine dielektrische Schicht angeordnet ist.

Erfindungsgemäß ist auf einer dem Trägermaterial abgewandten Seite zumindest eine Elektrode und/oder der dielektrischen Schicht zumindest stellenweise, zumindest eine, zumindest teilweise flüssigkeitsdurchlässige und/oder flüssigkeitsabsorbierende Feuchteschicht angeordnet, wobei somit die zumindest eine Elektrode und/oder die dielektrische Schicht in einer Querrichtung zwischen dem Trägermaterial und der Feuchteschicht angeordnet sind, sodass sich eine Kapazität durch die auf die dielektrische Schicht zumindest teilweise treffende Flüssigkeit zumindest teilweise verändert, wobei eine Verarbeitungseinheit diese Änderung misst und/oder speichert, sodass ein kapazitiver Feuchtesensor entsteht.

Dabei weist das oben beschrieben Verfahren die gleichen Vorteile und vorteilhaften Ausgestaltungen wie die obig beschriebene Vorrichtung auf.

Gemäß zumindest einer Ausführungsform umfasst das Verfahren zum Herstellen eines Textilstückes, insbesondere eines Kleidungsstückes, einen ersten Schritt, bei dem eine Vorgabeeinheit bereitgestellt wird, welche einer Form und/oder Größe oder einen Textilstoff vorgibt, wobei in einem nächsten Schritt der Textilstoff bereitgestellt wird, welche mit einem Textilbasisstoff gebildet ist.

In einem weiteren Schritt wird erfindungsgemäß mittels einer Produktionseinheit der Textilbasisstoff in zumindest zwei räumlich voneinander unabhängige und durch zumindest eine Schneidmarkierung getrennte Textilsegmente unterteilt, wobei die Textilsegmente nach deren Zusammenfügung das durch die Vorgabeeinheit vorgegebene Textilstück, insbesondere ein Kleidungsstück, ergeben, wobei in einem nächsten Schritt elektrisch leitfähige Fäden in die einzelnen Textilsegmente durch entsprechende Vorgabeparameter der Vorgabeeinheit eingearbeitet werden, wobei die elektrischen leitfähigen Fäden die Schneidmarkierung nicht kreuzen und auch nicht zumindest teilweise auf ihr verlaufen.

Gemäß zumindest einer Ausführungsform sind in der Vorgabeeinheit Mustersegmentunterteilungen hinterlegt, welche insbesondere von einem Benutzer ausgewählt werden und der Textilstoff nach Maßgabe dieser Mustersegmentunterteilung der verschiedenen Textilsegmente unterteilt wird.

Die Mustersegmentunterteilungen sind daher entweder in haptisch struktureller Form vorliegend, oder jedoch wiederum virtuell, das heißt lediglich datentechnisch in der Vorgabeeinheit hinterlegt und können entweder tatsächlich, das heißt strukturell, oder aber virtuell über die flächige Erstreckung des Textilstoffes gelegt werden, sodass sich dadurch eine Schneidschablone ergibt.

Gemäß zumindest einer Ausführungsform gibt der Benutzer die Form und/oder die Größe und/oder mit dem Textilstoff des zu fertigenden Textilstückes vor und diese Werte werden mit in der Vorgabe eindeutig hinterlegten entsprechenden Werten, insbesondere entsprechenden Mustersegmentunterteilungen, verglichen, wobei nach Übereinstimmung diese Werte die entsprechenden Mustersegmentunterteilung durch die Vorgabeeinheit ausgewählt wird.

Alternativ oder zusätzlich ist es möglich, dass bei einer Abweichung der vom Benutzer vorgegebenen Werte um höchstens, beispielsweise 25 %, ebenso entsprechende darauf passende Mustersegmentunterteilungen ausgewählt werden. Bei Überschreitung dieser Abweichung kann die Vorgabeeinheit vorzugsweise selbstständig nach einem alternativ passenden Mustersegment suchen. Sofern kein passendes Mustersegment vorhanden ist, ist denkbar, dass durch die dann von bekannten Mustern erheblich abweichenden Werte eine neue Mustersegmentunterteilung hinterlegt und erzeugt wird.
Gemäß zumindest einer Ausführungsform sendet die Vorgabeeinheit die entsprechende Mustersegmentunterteilung im Rahmen einer Datenkommunikation an die Produktionseinheit, wobei die Datenkommunikation eine Datenübertragung umfasst und weiter wobei die Datenübertragung auf einer Binärsprache oder Maschinensprache basiert. Das Mustersegment wird daher durch die Produktionseinheit strukturell haptisch oder aber rein virtuell visualisiert.

Gemäß zumindest einer Ausführungsform umfasst oder ist das Bereitstellen des Textilstoffes ein virtuelles oder technisches Laden von Daten des Textilstoffes in die Vorgabeeinheit.

"Virtuell" kann im Sinne der Erfindung bedeuten, dass ein eigentlich strukturell vorliegendes Element rein datentechnisch abgebildet wird.

Gemäß zumindest einer Ausführungsform werden auch die weiteren Produktionsschritte zumindest teilweise, vorzugsweise jedoch vollständig, lediglich virtuell durchgeführt und liegen daher lediglich in Form von Daten vor, sodass im Rahmen der Unterteilung des Textilstoffes und der Einarbeitung der elektrisch leitfähigen Fäden in den Textilstoff durch die Vorgabeeinheit und/oder durch die Produktionseinheit eine Produktionsdatei erstellt wird, welche alle relevanten für die Herstellung nötigen Daten beinhaltet.

Gemäß zumindest einer Ausführungsform sendet die Vorgabeeinheit und/oder die Produktionseinheit diese Produktionsdatei an eine Fertigungsmaschine zur haptischen Fertigung des Textilstückes, sodass die Fertigungsmaschine exakt entsprechend den Dateninhalten der Produktionsdatei das das Textilstück fertigt.

Spätestens daher in Zusammenhang mit der obig genannten Fertigungsmaschine wird ein strukturell haptisches Verfahren bereitgestellt.

Insbesondere sei jedoch erwähnt, dass die dann virtuellen Verfahrensschritte, welche jeweils von strukturellen Elementen (Vorgabeeinheit, Produktionseinheit) in technischer Hinsicht das technische Problem lösen, dass verschiedene Fertigungsvorgaben beispielsweise im Hinblick auf Form, Größe oder Beschaffenheit des Textilstoffes in vorzugsweise einer einzigen Produktionsdatei an eine Fertigungseinheit gesendet werden, sodass durch diesen vor der Fertigungsmaschine liegenden virtuellen Schrittkomplex nicht nur Arbeitsschritte gespart werden, sondern ebenso die Fertigungsmaschine an die individuellen technischen Bedürfnisse des Gesamtprozesses sowie an Bedürfnisse der Benutzers angepasst werden kann.

Bei der Vorgabeeinheit kann es sich um einen Rechner handeln, welcher einen Speicherchip und einen Rechnerchip, umfasst.

Entsprechendes kann für die Produktionseinheit gelten.

Gemäß zumindest einer Ausführungsform wird die Schneidmarkierung mit zumindest einem Schneidpunkt auf dem Textil festgelegt.

Auch dies kann strukturell haptisch oder aber auch virtuell getan werden. Bei den Schneidmarkierungen kann es sich um Lasermarkierungen, um Fahrtmarkierungen, um mechanische Markierungen, wie zum Beispiel Ritzen oder Lochen, handeln.

Gemäß zumindest einer Ausführungsform ist das Textilstück ausschussfrei gefertigt. Das heißt, dass auf einem einmal vorgegebenen Textilstück vorzugsweise 90 %, besonders bevorzugt 95 %, weiter bevorzugt 100 % der vorhandenen Fläche durch die obig erwähnten Textilsegmente gebildet ist. Mit anderen Worten umfasst ein derartiger Textilstoff keine von solchen Textilsegmenten freien Flächen (kein Ausschuss). Das Textilstück kann daher ausschussfrei gefertigt werden.

Des Weiteren betrifft die vorliegende Erfindung eine Vorrichtung zum Herstellen eines Textilstückes, insbesondere eines Kleidungsstückes, wobei die Vorrichtung zumindest eine Vorgabeeinheit, welche dazu eingerichtet und dafür vorgesehen ist, eine Form und/oder Höhe und/oder einen Textilstoff vorzugeben.

Erfindungsgemäß umfasst die Vorrichtung zumindest eine Produktionseinheit, welche dazu eingerichtet und dafür vorgesehen ist, dass der Textilbasisstoff in zumindest zwei räumlich voneinander unabhängige und durch zumindest eine Schneidmarkierung getrennte Textilsegmente unterteilt wird, wobei die Textilsegmente nach deren Zusammenfügung ein durch die Vorgabeeinheit vorgegebenes Textilstück, insbesondere ein Kleidungsstück, ergeben, und weiter wobei die elektrisch leitfähigen Fäden in die einzelnen Textilsegmente durch entsprechende Vorgabeparameter der Vorgabeeinheit einarbeitbar sind, wobei die elektrisch leitfähigen Fäden die Schneidmarkierungen nicht kreuzen und auch nicht zumindest teilweise auf ihr verlaufen.

Dabei weist die hier beschriebene Vorrichtung die gleichen Vorteile und vorteilhaften Ausgestaltungen auf, wie das obig beschriebene Verfahren und umgekehrt. Im Folgenden wird die vorliegende Erfindung anhand von drei Figuren näher beschrieben.

Dabei sind gleiche und gleich wirkende Bestandteile mit den gleichen Bezugszeichen versehen, auch wenn diese Bestandteile übertrieben groß dargestellt sein mögen.

Figur 1 zeigt ein grundsätzliches Verfahrensdiagramm zum Basisablauf eines hier beschriebenen Verfahrens.

Die Figur 2 zeigt ein detaillierteres Verfahrensdiagramm zum Ablauf des obigen Verfahrens gemäß der Figur 1.

Die Figur 3 zeigt in einer schematischen Draufansicht ein die auf Basis des Verfahrens der Figuren 1 und 2 erzeugte Segmentunterteilung des Textilstoffes vor dem Schneiden.

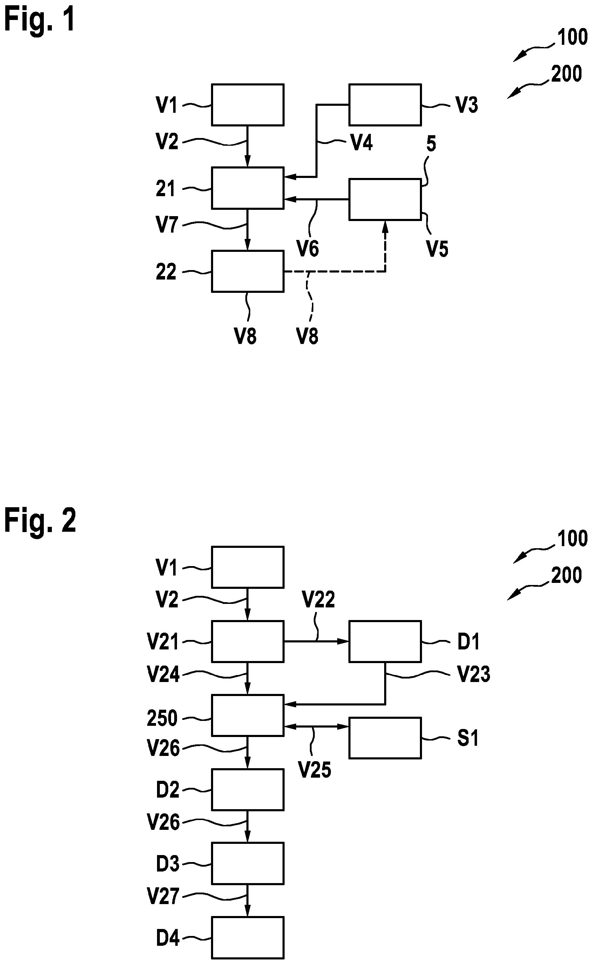

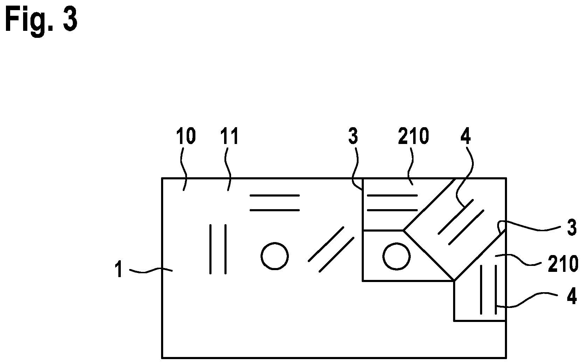

In der Figur 1 ist zunächst ein erster Verfahrensschritt V1 gezeigt, bei dem zunächst aus einer Datenbank das Material von elektrisch leitfähigen Fäden 4 (sichtbar in der Figur 3) sowie das Material eines Textilstoffes 10 eines Textilbasisstoffes 11 (sichtbar in der Figur 3= ausgewählt wird.

Diese werden im Rahmen eines Verfahrensschrittes V2 in eine Vorgabeeinheit 21 eingespeist. Gleichzeitig werden in die Vorgabeeinheit 21 von dem Benutzer vorgegebene Parameter zur Herstellung des von ihm gewünschten Textilstückes 1 ebenso in die Vorgabeeinheit 21 eingeladen.

Dies beinhaltet daher die Schritte V3 und V4.

In einem Schritt V5 werden bestimmte Maschinenbedingungen sowie Maschineneigenschaften einer Fertigungsmaschine 5 ebenso in die Verarbeitungseinheit 21 geladen. Mit anderen Worten verfügt daher die Verarbeitungseinheit 21 gemäß der Schritte V1 bis V6 über alle relevanten Daten zur Herstellung des Textilstückes 1.

Die Verarbeitungseinheit 21 verarbeitet nun diese Daten und erstellt somit ein an das Textilstück 1 angepasstes Produktionsprogramm mittels eines Schrittes V7, sodass in einem Schritt V8 das Produktionsprogramm von einer Produktionseinheit ebenso an eine weitere oder die Fertigungsmaschine 5 gesendet werden kann. Alternativ hierzu ist jedoch auch möglich, dass die Programmdatei an jede andere Produktionsmaschine oder Fertigungsmaschine gesendet werden kann.

Mit anderen Worten ist in der Figur 1 ein, vorzugsweise rein, virtuelles Erstellungsprogramm dargelegt, welches jedoch in technischer Hinsicht zwingend nötig ist, um die Fertigungsmaschine 5 betreiben zu können. Zudem ist das Produktionsprogramm in technisch haptischer Hinsicht in der Verarbeitungseinheit 21 nicht nur hinterlegt, sondern wird durch diese erst erzeugt.

In der Figur 2 ist ein innerer Datenverarbeitungsablauf innerhalb der Vorgabeeinheit näher dargestellt. Wiederum dargestellt sind die Schritte V1 und V2, wobei nunmehr erkannt werden kann, dass mittels eines Schrittes V21 eine Datenintegration aller durch die in der Figur 1 gezeigten Daten zunächst durchgeführt wird. Ebenso erkannt werden kann, dass diese Daten dann verarbeitet werden, wobei in einem Schritt V22 eine Designsimulation oder eine Designverifikation D1 stattfinden kann, bevor nach einer entsprechenden Designsimulation/ - verifikation im Rahmen eines Schrittes V23 diese Daten wieder in eine Verarbeitungsbox 250 eingespeist werden.

Alternativ oder zusätzlich werden nach dem Schritt der Datenintegration mittels eines Schrittes V24 diese Daten ebenso in die Verarbeitungsbox 250 eingespeist. Die Verarbeitungsbox 250 verarbeitet und erzeugt die entsprechenden Schneidmarkierung 3 (sichtbar in der Figur 3) auf dem jeweils zugrunde liegenden Textilbasisstoff 11 und gleicht die dann gewonnenen Designdaten mittels eines Schrittes V25 im Rahmen einer Simulation/Verifikation S1 mit einem gewünschten Designcutprofil ab. Dieses Designcutprofil kann wiederum in der Verarbeitungseinheit 21 hinterlegt sein.

Sofern etwaige Abweichungen von einem gewünschten Designcutprofil festgestellt werden, kann in einem Schritt V26 dieses Design an ein optimales oder gewünschtes oder in der Verarbeitungseinheit 21 hinterlegtes Designprofil D2 angepasst werden. In einem Schritt V26 wird ein Designruleheck D3 durchgeführt, wobei in einem Schritt V27 dann letztendlich eine Programmdatei D4 erzeugt wird.

In der Figur 3 ist schematisch ein Textilbasisstoff 11 dargestellt, welcher in die unterschiedlichen Textilsegmente 210 jeweils unterteilt ist. Ebenso erkannt werden kann, dass innerhalb der Textilsegmente 210 entsprechende leitfähige Fäden 4 eingewoben sind oder aber diese elektrisch leitfähigen Fäden 4 selbst einen Textilfaden des Textilstoffes 11 sogar ersetzen.

Des Weiteren ist entscheidend, dass keiner der elektrisch leitfähigen Fäden 4 die Schneidmarkierungen 3 durchschneiden oder auf ihnen liegen, sodass die auch nach dem entsprechenden Schneiden entlang der Schneidmarkierungen 3, beispielsweise mittels eines Lasers oder eines Messers, elektrisch leitfähigen Fäden 4 keine Schnittflächen aufweisen.

Die Anmelderin behält sich vor, sämtliche in den Anmeldungsunterlagen offenbarten Merkmale als erfindungswesentlich zu beanspruchen, sofern sie einzeln oder in Kombination gegenüber dem Stand der Technik neu sind.

### Bezugszeichenliste

- 1: Textilstück
- 3: Schneidmarkierung
- 4: elektrisch leitfähige Fäden
- 5: Fertigungsmaschine
- 10: Textilstoff
- 11: Textilbasisstoff
- 21: Verarbeitungseinheit
- 100: Verfahren
- 200: Vorrichtung
- 210: Textilsegmente
- 250: Verarbeitungsbox

- D2: Designprofil
- D3: Designrulecheck
- D4: Programmdatei
- S1: Simulation/Verifikation
- V1: Verfahrensschritt
- V2: Verfahrensschritt
- V3: Verfahrensschritt
- V4: Verfahrensschritt
- V5: Verfahrensschritt
- V6: Verfahrensschritt
- V7: Verfahrensschritt
- V8: Verfahrensschritt
- V21: Verfahrensschritt
- V22: Verfahrensschritt
- V23: Verfahrensschritt
- V24: Verfahrensschritt
- V25: Verfahrensschritt
- V26: Verfahrensschritt
- V27: Verfahrensschritt

## Patentansprüche

1. Verfahren (100) zum Herstellen eines Textilstückes (1), insbesondere eines Kleidungsstückes, umfassend die folgenden Schritte:
- Bereitstellen einer Vorgabeeinheit, welche eine Form und/oder Größe und/oder einen Textilstoff (10) vorgibt,
∘ Bereitstellen des Textilstoffes (10), welcher mit einem Textilbasisstoff (11) gebildet ist, wobei dieser Textilbasisstoff (11) entweder strukturell oder virtuell zusammen mit elektrisch leitfähigen Fäden, gewebt wird, sodass die elektrisch leitfähigen Fäden nicht erst nachträglich in den Textilbasisstoff eingebracht werden, sondern bereits während eines Webprozesses in den Textilbasisstoff (11) eingewoben werden,
**dadurch gekennzeichnet, dass**
- in einem nächsten Schritt mittels einer Produktionseinheit der Textilbasisstoff (11) in zumindest zwei räumlich voneinander unabhängige und durch zumindest eine Schneidmarkierung (3) getrennte Textilsegmente (210) unterteilt wird, wobei die Textilsegmente (210) nach deren Zusammenfügung das durch die Vorgabeeinheit vorgegebene Textilstück (1), insbesondere ein Kleidungsstück, ergeben.

2. Verfahren (100) nach Anspruch 1,
**dadurch gekennzeichnet, dass**
in der Vorgabeeinheit Mustersegmentunterteilungen hinterlegt sind, welche, insbesondere von einem Benutzer, ausgewählt werden, und der Textilstoff (10) nach Maßgabe dieser Mustersegmentunterteilung in die verschiedenen Textilsegmente (210) unterteilt wird.

3. Verfahren (100) nach Anspruch 1 oder 2,
**dadurch gekennzeichnet, dass**
der Benutzer die Form und/oder die Größe und/oder den Textilstoff des zu fertigenden Textilstückes (1) vorgibt, und diese Werte mit in der Vorgabeeinheit hinterlegten entsprechenden Werten, insbesondere entsprechenden Mustersegmentunterteilungen, verglichen werden, wobei nach Übereinstimmung dieser Werte die entsprechende Mustersegmentunterteilung durch die Vorgabeeinheit ausgewählt wird.

4. Verfahren (100) nach dem vorhergehenden Anspruch,
**dadurch gekennzeichnet, dass**
die Vorgabeeinheit die entsprechende Mustersegmentunterteilung im Rahmen einer Datenkommunikation an die Produktionseinheit sendet, wobei die Datenkommunikation eine Datenübertragung umfasst, und weiter wobei die Datenübertragung auf einer Binärsprache und/oder einer Maschinensprache basiert.

5. Verfahren (100) nach zumindest einem der vorhergehenden Ansprüche,
**dadurch gekennzeichnet, dass**
das Bereitstellen des Textilstoffs (10) ein virtuelles und datentechnisches Laden von Daten des Textilstoffes in die Vorgabeeinheit umfasst oder ist.

6. Verfahren (100) nach dem vorhergehenden Anspruch,
**dadurch gekennzeichnet, dass**
auch die weiteren Produktionsschritte zumindest teilweise, vorzugsweise jedoch vollständig, lediglich virtuell durchgeführt werde und daher lediglich in Form von Daten vorliegen, sodass im Rahmen der Unterteilung des Textilstoffes (10) und der Einarbeitung der elektrisch leitfähigen Fäden (4) in den Textilstoff (10) durch die Vorgabeeinheit und/oder durch die Produktionseinheit eine Produktionsdatei erstellt wird, welche alle relevanten für die Herstellung nötigen Daten beinhaltet.

7. Verfahren (100) nach dem vorhergehenden Anspruch,
**dadurch gekennzeichnet, dass**
durch die Vorgabeeinheit und/oder durch die Produktionseinheit diese Produktionsdatei an eine Fertigungsmaschine (5) zur haptischen Fertigung des Textilstückes (1) gesendet wird, sodass die Fertigungsmaschine (5) exakt entsprechend den Dateninhalten der Produktionsdatei das Textil fertigt.

8. Verfahren (100) nach dem vorhergehenden Anspruch,
**dadurch gekennzeichnet, dass**
die Schneidmarkierung (3) mit zumindest einem Schneidpunkt auf dem Textil festgelegt wird.

9. Verfahren (100) nach dem vorhergehenden Anspruch,
**dadurch gekennzeichnet, dass**
das Textilstück (1) ausschussfrei gefertigt wird.

10. Vorrichtung (200) zum Herstellen eines Textilstoffes (10), insbesondere eines Kleidungsstückes, umfassend:
zumindest eine Vorgabeeinheit, welche dazu eingerichtet und dafür vorgesehen ist eine Form und/oder Größe und/oder einen Textilstoff (10) vorzugeben
**gekennzeichnet durch**
zumindest eine Produktionseinheit, welche dazu eingerichtet und dafür vorgesehen ist, dass der Textilbasisstoff (11) in zumindest zwei räumlich voneinander unabhängige und durch zumindest eine Schneidmarkierung (3) getrennte Textilsegmente (210) unterteilt wird, wobei die Textilsegmente (210) nach deren Zusammenfügung ein durch die Vorgabeeinheit vorgegebenes Textilstück (1), insbesondere ein Kleidungsstück, ergeben, und weiter wobei Bereitstellen des Textilstoffes (10), welcher mit einem Textilbasisstoff (11) gebildet ist, wobei dieser Textilbasisstoff (11) entweder strukturell oder virtuell zusammen mit elektrisch leitfähigen Fäden, gewebt ist, sodass die elektrisch leitfähigen Fäden nicht erst nachträglich in den Textilbasisstoff eingebracht werden, sondern bereits während eines Webprozesses in den Textilbasisstoff (11) eingewoben wurden.
